# EUROPEAN PATENT SPECIFICATION

(11) **EP 4 142 698 B1**
(45) Date of publication and mention of the grant of the patent: **28.02.2024**
(21) Application number: 21721188.7
(22) Date of filing: 15.04.2021
(51) Int. Cl.: A61K 9/20, A61K 31/40, A61K 31/4985, A61K 31/505, A23K 10/18, A23K 10/22, A23K 10/30, A23K 10/33, A23K 20/163, A23K 50/50, A01N 25/08, A01P 11/00

(54) **SEMI-SOLID FORMULATION FOR VOLUNTARY ORAL ADMINISTRATION OF BIOACTIVE COMPOUNDS TO RODENTS**
HALBFESTE FORMULIERUNG ZUR FREIWILLIGEN ORALEN VERABREICHUNG BIOAKTIVER VERBINDUNGEN AN NAGETIERE
FORMULATION SEMI-SOLIDE DESTINÉE À L'ADMINISTRATION ORALE VOLONTAIRE DE COMPOSÉS BIOACTIFS À DES RONGEURS

(30) Priority: 27.04.2020 PT 2020116301
(43) Date of publication of application: 08.03.2023
(73) Proprietor: Universidade de Coimbra, 3004-531 Coimbra (PT); Instituto Politécnico de Coimbra, 3000-271 Coimbra (PT)
(72) Inventor: DOMINGUES VIANA, Sofia Andreia, 3030-482 Coimbra (PT); FERNANDES REIS, Flávio Nelson, 3030-218 Coimbra (PT)
(74) Representative: Couto, Cláudia
(86) International application number: PCT/IB2021/053124
(87) International publication number: WO 2021/220099

(56) References cited:
- EP-A1- 0 772 391
- US-A1- 2005 181 003
- DHAWAN SANDEEP S ET AL: "Oral dosing of rodents using a palatable tablet", PSYCHOPHARMACOLOGY, SPRINGER VERLAG, BERLIN, DE, vol. 235, no. 5, 6 March 2018 (2018-03-06) , pages 1527-1532, XP036490470, ISSN: 0033-3158, DOI: 10.1007/S00213-018-4863-2 [retrieved on 2018-03-06] cited in the application
- RAYA JULIANA ET AL: "Corticosterone Assimilation by a Voluntary Oral Administration in Palatable Food to Rats", JOURNAL OF APPLIED ANIMAL WELFARE SCIENCE, vol. 22, no. 1, 11 May 2018 (2018-05-11), pages 37-41, XP055817614, US ISSN: 1088-8705, DOI: 10.1080/10888705.2018.1471605

## Description

### Technical field

This application relates to a formulation for voluntary oral administration of bioactive compounds to rodents.

### Background art

Oral route is the preferred method for drug administration (e.g. tablet, capsule, syrup) to humans (Stewart et al. 2016). During drug development, the major pre-clinical tools for new pharmacological agent screening prior to clinical testing often involves laboratory animal experimentation and distinct strategies for oral drug administration to rodents have been described. Gavage is the most straightforward approach to achieve precise enteric drug dosing in mice and rats (Abelson et al. 2012). Briefly, this methodology involves the insertion of a flexible catheter or a rigid needle with a ball tip through the mouth into the esophagus with direction to the stomach. Nevertheless, gavage comprises a sequence of traumatic procedures that i) lead to wounding of esophagus or stomach and related complications (e.g. gastroesophageal aspiration and pulmonary injury; inability to swallow; asphyxia due to impacted food and bedding material in the oropharynx and, ultimately, mortality); ii) elicit a significant distress response, in a vehicle- and dose-volume dependent fashion, arising from the removal of the animal from its cage and subsequent manual restraint, even in trained animals; iii) require substantial technical skills (e.g. oropharyngeal anatomy knowledge) (Brown, Dinger, and Levine 2000; Jones, Boyd, and Wallace 2016). Moreover, trauma associated with force-fed gavage can significantly alter physiological parameters (e.g. heart rate, blood pressure, plasmatic corticosterone/fecal corticosterone metabolites) that are crucial in a wide array of preclinical studies of several pharmacological agents (e.g. cardiovascular, endocrine and/or central nervous system bioactive modulators), introducing bias into experimental results (Brown, Dinger, and Levine 2000; Diogo et al. 2015; Vandenberg et al. 2014; Yardley et al. 2015). Overall, oral gavage is not a suitable methodology for long-term/chronic and repetitive treatments that are often required during the drug discovery phase regarding the chronic nature of most diseases (e.g. diabetes, hypertension, neurodegenerative diseases), creating a challenge for drug testing in preclinical assays (Gonzales et al. 2014). This reason often leads researchers to give up from oral bioactive compounds administrations choosing alternative administration routes (e.g. intraperitoneal, subcutaneous), bearing in mind that these strategies may narrow workplan endpoints and further translation to the clinical ground. Hence, non-invasive and stress-free methods for force-fed gavage of oral bioactive compounds administration into rodents have been an emergent area in the scientific community and pharmaceutical field (Diogo et al. 2015; Gonzales et al. 2014; Küster et al. 2012; Allen et al. 2015). Accordingly, distinct "least-stressful" alternatives (when compared to gavage procedure) for oral drug delivery to rodents have been described:
**Alternative 1.** Drug suspension in sucrose solution (5% - 10%) and voluntary syringe feeding (Atcha et al. 2010; Schleimer, Johnston, and Henderson 2005);
**Alternative 2.** Fast dissolving oral biofilm containing drug and voluntary syringe feeding ( Allen et al. 2015; Huynh et al. 2016; Yardley et al. 2015);
**Alternative 3.** Drug given in the diet (food/drinking water) (Molina-Cimadevila et al. 2014; Küster et al. 2012);
**Alternative 4.** Mealworms injected-drug (Sobolewski et al. 2016) ;
**Alternative 5.** Drug admixed with palatability-improving substances (premixed drug-chocolate paste/ sugar paste/ peanut butter/ honey/ flavored dough/ flavored gelatins) (Abelson et al. 2012; Gonzales et al. 2014; Huang-Brown and Guhad 2002; Liles et al. 1998; Taylor et al. 2016; Walker et al. 2012; Zhang and Zhang 2011).

Yet, these strategies are still associated with important disadvantages that may plight experimental endpoints. Regarding drug syringe feeding approaches (Alternative 1 and 2), these are not "truly-voluntary" alternatives considering that animals need to be restrained by the operator for drug administration. This is an obvious disadvantage as animal removal from its cage and subsequent restraint display a huge amount of human interference with experimental animal and inherent distress. The remaining methodologies (Alternative 3-5) are truly voluntary due to animal willingness to freely choose drug ingestion without operator's intervention. Yet, there are still critical issues that need to be improved. The first one is linked with animal housing as most of these procedures require individually housing to ensure correct dosage and to confirm complete ingestion of formulation. Since rodents are social animals typically paired- or grouped-house, disruption to their social hierarchy by single housing can be highly stressful, leading to behavioral/physiological changes that may compromise their well-being (ethical constrains) and potentially confound experimental endpoints. Drug given in the food and/or water of grouped-house animals (Alternative 3) overcomes this social/hierarchy constraint, but under- or overdosing may occur, and precise drug dosing is not achieved. Regarding the group-housed mealworms-injected drug protocol (Alternative 4), albeit innovative, it is generally dubious in distinct perspectives since: i) it is unable to guarantee inactive warm metabolism and preservation of chemically-unaltered drugs for rodent administration; ii) is somehow arguable as it may not fulfill laboratory animal facility safety issues; iii) is largely hardworking and timeconsuming.

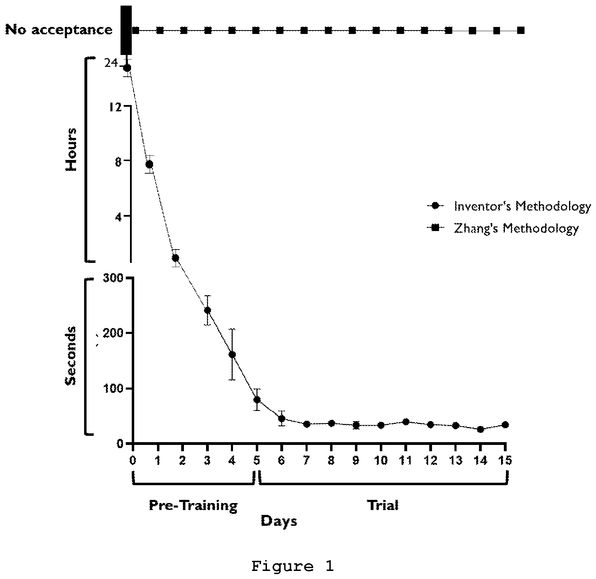

A second important topic relies on palatability-improving substances added to drug vehicles to improve animal cooperation (Alternative 5). These compounds may substantially alter nutritional status and critically disrupt animal metabolic pathways (e.g. blood glucose levels/serum lipid profile), namely when long-term regimens are required (Diogo et al. 2015). An interesting methodology that tried to overcome this inconvenience is the flavored modified "Jelly pill", proposed by Zhang (2011), who incorporated a non-caloric sweetener (sucralose) instead of sucrose into the formula, making it suitable for chronic oral drug delivery minimizing animal metabolic disruption. In an attempt to replicate this protocol, our group was able to successfully manufacture the Zhang's jelly pill (Fig 1). However, voluntary acceptance (i.e., dosage form voluntarily taken into the mouth of the animal) of Zhang's jelly pill was completely null within 14 days of consecutive 24 hours of exposition in distinct batches of overnight fasted males and females C57BL/6 mice. Animals showed an exploratory behavior by pushing the pill into the wall cages, but always avoided direct contact within consecutive 14 days attempts. Upon physical contact with the formula, animals displayed high rates of grooming behavior, denoting severe formulation constraints that is likely to subsist Zhang's jelly pill animal rejection. The present inventors are confident that rodent's avoidance of contact suggests that, despite Zhang's vehicle appealing palatable features, the sticky surface of the vehicle deters the animal's from holding it. Thus, the inventors recognize the importance of palatability to invite a first approach and texture's crucial role to ensure the animal's voluntary complete consumption. Consistently, robust evidence of successful application of Zhang's methodology are lacking and modified formulas have been already reported, hinting for a lack of reproducibility (Vidal-Martinez et al. 2016, Dhawan, S. et al, 2018). Importantly, modifications proposed by aforementioned authors also present drawbacks since i) incorporates excipients that may alter metabolic profile (e.g. bacon soft pellets) and ii) requires individual housing for pill administration.

Globally, chief technical concerns of effective alternative protocols for oral bioactive compounds delivery in laboratory rodents are still warranted, in line with similar efforts of optimized patented formulations for veterinary drug administration with therapeutic purposes (Allen et al. 2015; Derrieu et al. 2014; Kalbe et al. 2004; Larry et al. 1992; Thombre & Kasraian, 2003; Thombre, A., 2005). Analogous difficulties on rodents' toxicants voluntary consumption are described as well. In fact, rodenticides formulations (e.g. pellets, dust, loose grains, pastes) are currently prepared with active ingredients like fats, seeds and palatability enhancers that entice the voluntary consumption of the toxicant. Finished baits containing the toxicant must avoid bait shyness and aversion, increasing their voluntary consumption and effectiveness.

Taking into account the above deficiencies of bioactive compounds oral administration to rodents, the present invention proposes a new approach mimicking the single-dose pill administration method widely used in human clinical practice. The main goal relied on the optimization of an easy, safe, consistent, precisely dosed and efficient formulation for voluntary rodent oral drug administration. Former authors already emphasized the plentiful of advantages arising from the pill dosing method, namely: i) allows for individual precise dosing (amount of drug/body weight); ii) requires small amounts of drug (when compared to drugs admixed in the food or drinking water); iii) overcomes drug hydrophilicity/lipophilicity requirements for vehicle-solubilization; iv) does not elicit significant stress responses; v) is efficient for repetitive and long-term delivery of bioactive compounds into rodents (Kopf et al. 2010; Walker et al. 2012; Zhang and Zhang 2011) . However, previous pill-dosing formulas for rodents are still questionable in terms of i) metabolic pathways disruption in the context of preclinical assays, ii) reproducibility and iii) laboratory animal housing and welfare. Besides the lack of reproducibility observed with Zhang's approach, the pill formula proposed by Walker and colleagues (2012) is based on a bacon-flavored dough diet with high protein and fat levels and, consequently, with probable long-term outcomes on animal metabolic profile. In addition, the formula recently proposed by Dhawan, S. et al (2018) is based on fruit juice concentrates to enhance palatability regardless of the very likely deleterious interactions between juice components and the bioactive compounds intended to administer. Hence, the present inventors have developed a new pill formula for rodent's voluntary acceptance that overcomes previous drawbacks.

### Summary

The present patent application discloses herein a semi-solid formulation for voluntary oral administration of bioactive compounds to rodents.

The semi-solid pill formulation disclosed herein comprises:
- at least one thickening agent, wherein said thickening agent is selected from gelatin, agar, carrageenan, sodium alginate and pectin;
- a digestible sweetener, wherein the digestible sweetener is selected from sucralose and calcium saccharin;
- a diluent agent, wherein the diluent agent is a powder with a granulometry between 300 and 800pm,obtained from finely grounded seeds , cereal grains or cereal grain based diet for rodents, in a concentration between 10 and 30% m/v;
- at least one flavor masking agent;
- a bioactive compound.

In one embodiment, the finely grounded seeds are selected from at least one of sunflower and canary.

In one embodiment, the finely grounded cereal grain based diet for rodents comprise at least one of dehulled soybean meal, wheat middlings, flaked corn, ground corn, fish meal, cane molasses, ground wheat, dried whey, soybean oil and brewers dried yeast.

In one embodiment, the formulation further comprises a preservative.

In one embodiment, the thickening agent is gelatin in a concentration between 5 and 20% m/v.

In another embodiment, the thickening agent is agar in a concentration between 1 and 5% m/v.

In another embodiment, the thickening agent is carrageenan in a concentration between 0.02 and 3% m/v.

In another embodiment, the thickening agent is sodium alginate in a concentration between 0.7 and 2% m/v.

In another embodiment, the thickening agent is pectin in a concentration between 0.5 and 5% m/v.

In one embodiment, the non-digestible sweetener is sucralose in a concentration between 1 and 5% m/v.

In another embodiment, the non-digestible sweetener is calcium saccharin in a concentration between 0.1 and 0.25% m/v.

In one embodiment, the flavor masking agent is selected from vanilla, strawberry, blueberry and chocolate.

In one embodiment, the formulation of the present patent application is for use in the oral administration of drugs and bioactive compounds to rodents.

In another embodiment, the formulation of the present patent application is used in toxicological screening methods of rodents.

In another embodiment, the formulation of the present patent application is used in the production of toxicant baits for rodents.

### Disclosure/Detailed Description

The present patent application discloses herein a semi-solid formulation for voluntary oral administration of bioactive compounds to rodents.

Taking into account the above deficiencies of the prior art approaches described above for the oral administration of bioactive compounds to rodents, the present patent application discloses a new approach mimicking the single-dose pill administration method widely used in human clinical practice. The main goal relies on the optimization of an easy, safe, consistent, precisely dosed and efficient formulation for voluntary rodent oral drug administration through the pill dosing method. It is important to emphasize the plentiful of advantages arising from the use of the presently disclosed formulation: i) allows for individual precise dosing (amount of drug/body weight); ii) requires small amounts of drug (when compared to drugs admixed in the food or drinking water); iii) overcomes drug hydrophilicity/lipophilicity requirements for vehicle-solubilization; iv) does not elicit significant stress responses; v) is efficient for repetitive and long-term delivery of bioactive compounds into rodents. Thus, the new semi-solid pill formulation disclosed herein is designed to i) be a carrier of bioactive compounds to rodents; ii) achieve high acceptance index and voluntary oral consumption in a reproducible fashion and iii) to preserve metabolic profile and welfare of experimental laboratory rodents. Overall, the present invention proposes a new approach for voluntary rodent oral bioactive compounds administration through a single-dose pill methodology able to achieve voluntary acceptance, mimicking what happens with solid pharmaceutical compositions widely used in human clinical practice. This invention relates to a new semi-solid pill formula that is a suitable carrier of drugs, toxins, biomass (e.g. vegetable origin), vaccines and/or adjuvants and other bioactive compounds, being appealing for pharmaceutical, veterinary and food supply industrial sectors as well as for biomedical institutes and worldwide academies.

To achieve pill's voluntary acceptance without significant effects on metabolic homeostasis and distress responses, it is paramount to consider innate rodent's investigatory behaviors, neophobia and feeding preferences, namely those topics related with i) flavor, ii) texture and iii) size. It is generally recognized, namely from available rodent bait formulas, that sugars, animal fats and cereals are effective additives to improve acceptance and palatability. However, chronic delivery of high contents of fats and digestible sugars may significantly impact rodent's metabolism overtime and therefore interfere with pre-clinical research goals. To circumvent these issues, the formula presented herein incorporates low percentages of sweeteners (e.g. sodium saccharine, sucralose) to enhance rodent's voluntary acceptance. Since sweeteners selected herein are non-digestible sugars (avoiding the introduction of calories into the formula), it does not stimulate insulin/incretin release nor alter gastric emptying effects, making this vehicle suitable to preserve animal metabolic parameters. Trace amounts of essences are concomitantly used to provide a taste masking effect for the common unpleasant flavors arising from admixed bioactive compounds. Various flavors are available, namely vanilla, strawberry, blueberry, chocolate but not limited to other scents that can incite rodent feeding behavior. A two-bottle test has been performed to measure the preference for strawberry and chocolate essences, with no significant differences recorded between the two flavors.

A second critical aspect relies on pill texture and consistency. The inventors found that, despite rodents innate investigatory behavior to previous formulas (e.g. Zhang's methodology), a soft and non-sticky texture is warranted for pill acceptance and complete consumption. Hence, the innovative character of present invention relies on texture optimization to decrease the stickiness sensation. Formula presented herein incorporates edible texturizing agents (thickening and diluent agents) in optimal amounts to modify the overall pill texture towards a decreased stickiness and optimal semi-solid consistency, thus achieving a preferred nonadherent tactile experience that improves rodent's acceptance. Edible thickening agents (e.g. gelatin, pectin, agar agar) help to modify the pill's sensory features in terms of mechanical solid properties (texture) and flow behavior (viscosity). The formula presented herein preconizes the use of one of these agents in the lowest amount required to achieve a semi-solid matrix while minimizing the sticky texture of the final product. In addition, finely grounded seeds/cereals blends were chosen as palatable diluent agents, not only to support the homogeneous incorporation of the bioactive compounds within the vehicle, but foremost, because they decrease pill's adhesive texture that deters rodent's acceptance. We surveyed several palatable diluents (e.g. sunflower seeds, canary seeds, cereal grain and cereal grain based standard rodent's diets) and observed rodent's voluntary acceptance in a reproducible way. In addition, we established that particles size between 300-800pm corresponds to the optimal granulometry for diluents, providing not only a non-sticky texture but also allowing pill waste minimization once it is consumed. Palatable seeds/cereals blends incorporated in the present formula are also key elements to avoid rodent's neophobic behavior towards pill investigation and complete consumption. A third major issue of this invention relies on pill size. Since rodent's voluntary acceptance is the foundation for pill consumption, pill size should cope with the usual feeding habits of rodents. Optimal pill size preconized in the present invention (diameter ≅ 0.6-0.7 cm; height ≅ 0.4-0.6 cm).

The formulations of the present invention are prepared by mixing a palatable diluent agent with two palatability improving agents - flavouring essence and non-digestible sugar - to obtain a flavored, sweetened and non-sticky semi-solid matrix that accomplish the oral voluntary acceptance goal. Palatability improving agents utilized in the present invention are all non-toxic, food acceptable, do not affect bioactive compound with which they are associated and are used in small amounts to minimize expenses and maximize production efficiency.

Bioactive compounds suitable for use with the semi-solid vehicle disclosed herein include drugs, toxins and/or biomass or any combination thereof, but do not limit the scope of the invention composition. The amount of bioactive agent(s) varies and is dependent on the required dosage for treatment. The amount of semi-solid vehicle is the delivery effective amount that can be determined for any bioactive compound. Preservatives that can be employed are compounds conventionally used for pharmaceutical preparations and foodstuffs. Suitable, but non-limiting examples are benzoic acid, derivative salts (e.g. calcium, sodium) and esters (e.g. methyl p-hydroxybenzoate, ethyl p-hydroxybenzoate, propyl p-hydroxybenzoate), sorbic acid, fumaric acid, lactic acid, citric acid and derivative salts (e.g. sodium citrate), propyl gallate, citric acid, ascorbic acid, ascorbin palmitate, tocopherol, tocopherol acetate, butylhydroxytoluene and butylhydroxyanisole. Semi-solid pill formula and nutritional composition are presented in Table 1 and 2, respectively.

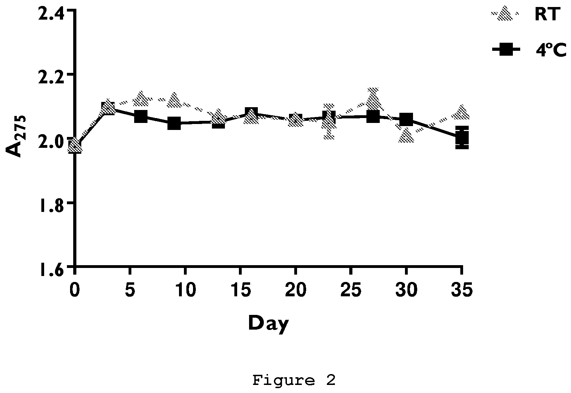

During the manufacturing process, the foundation for the semi-solid pill formulation is a thickening agent and a non-digestible sugar solution, prepared in an erlenmeyer conical flask immersed in a water bath on a stirring hot plate (Caution: heat up to 35-40°C with cap loosely screwed on). This solution can be aliquoted in conical-bottom disposable plastic tubes and stored at -20°C for months until reuse. The following steps illustrates the manufacture protocol: the flavouring essence, diluent agent and preservatives are incorporated into the thickening agent/non-digestible sugar solution and stirred for proper mixing (Important: leave the vehicle immersed in the water bath at 35-40°C, with constant stirring, until next steps) . To formulate a batch of 96 semi-solid pills, a 96-well plate round bottom (with lid) is used as a pill mold base plate to produce a semi-solid with uniform size, shape and weight, as depicted in. Each well is filled with a desired volume of vehicle, usually normalized according to animal species and body weight, the plate is covered with a lid and stored at 4°C. For bioactive compound delivery, a precise amount of the active ingredient (usually calculated in terms of rodent's body mass) is previously weighted/measured and transferred into each well. Then, the desired volume of vehicle is quickly admixed in the well with a stainless-steel stirring rod. This procedure results in active ingredient suspension rather than solubilization, circumventing solubility concerns. Alternatively, bioactive compound(s) can be admixed into the vehicle before mold base transfer. The volume considered needs to be small enough to ensure the consumption of the entire piece of the semi-solid pill by rodents (e.g. ≈ 100µl in C57BL/6 mice) but simultaneously adequate to incorporate the required amount of drug. Bioactive compounds in a liquid form may be incorporated as well. Semi-solid pills become ready semi-solid (4°C). They retain a semi-solid texture at room temperature (RT < 40-50°C) and can be stored at 4°C or RT depending on the preservatives included, as exemplified in Fig 2. Hence, semi-solid pills carrying bioactive compounds can be warehoused at both 4^{a}C or RT for months depending on the intrinsic stability properties of active ingredient(s) and preservatives added, similarly to what happens with other commercially available pharmaceutical and/or food products for human consumption. The protocol presented herein can be easily scaled-up to an industrial setting.

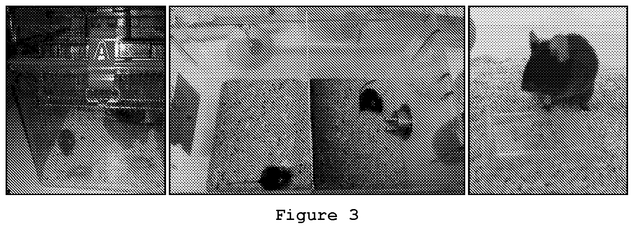

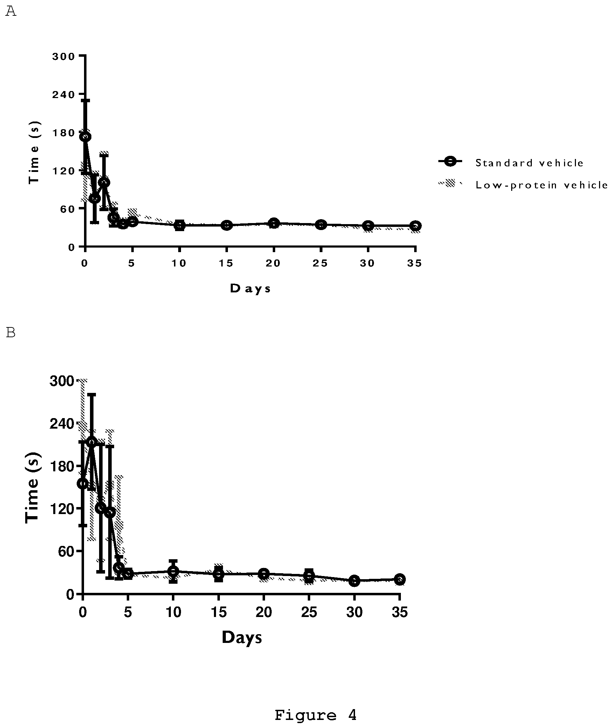

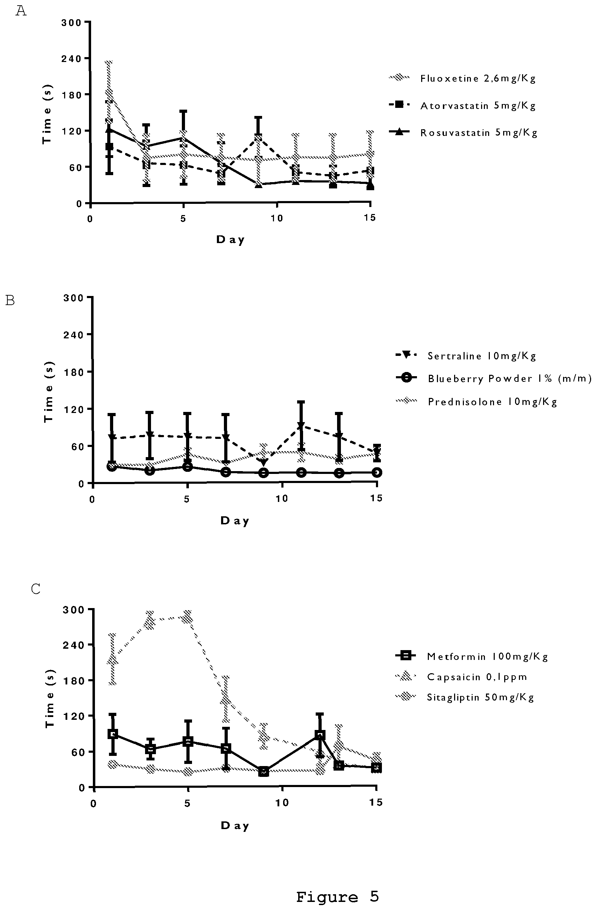

The methodology underlying semi-solid pill oral drug administration to laboratory rodents was conceptualized to ensure animal welfare maintenance during bioactive compounds delivery. This protocol requires a brief training period (3-5 days of overnight/morning fasting) to eliminate the normal neophobic behavior of laboratory rodents to a novel item (e.g. bury the pill with bedding material). In order to ensure that each animal completely ingests the semi-solid pill, a division is introduced into the cage only at the moment of administration. Semi-solid pills are introduced in each side of the cage in disposable plastic trays (Fig 3). Following total consumption, the division is removed and animals reestablish physical contact. After a pill is placed into a cage, rodents quickly investigate it and typically consumed it in less than one minute (Fig. 4), the most preferred "voluntary acceptance index" (Thombre & Kasraian, 2003). This high acceptance rate remained, at least, for 35 days (equivalent to ≈3.3 years in humans), compatible with chronic regimens of drug delivery (Sengupta P., 2013). To provide unequivocally evidence of semi-solid pill ingestion following bioactive compounds incorporation, a battery of anti-diabetic/dyslipidemic/depressants/inflammatory as well as a vegetable biomass were tested for voluntary consumption. All tested substances were found to accomplish the optimal index of voluntary acceptance when administered in the present invention, including the organoleptic unpleasant capsaicin (a rodent repellent), the molecule responsible for the spicy taste in most food ingredients (Fig. 5). Overall, this semi-solid pill formulation minimizes negative effects of previous formulations and methodologies of preclinical rodent experimentation.

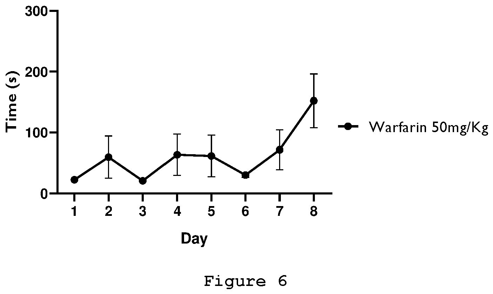

Aside from experimental rodent experimentation, this new semi-solid pill vehicle is compatible with the widest toxins used for domestic rodents' infestations, as the high voluntary acceptance of warfarin (Lim G., 2017) clearly demonstrates (Fig 6). Rodents displayed a poor health status and evident signs of toxicity from day 4 post-administration and were euthanized at day 8 regarding the critical signs of illness (e.g. bleeding), in accordance with good practices guidelines provided by European Community directive (2010/63/EU). In opposition to the neophobic behavior of laboratory rodents to a novel item, wild rodents display innate food demand and hunter skills that circumvent the pre-training phase required for laboratory experimentation. Considering semi-solid vehicle rodents' voluntary readily consumption, this formula is suitable for domestic toxicants baits.

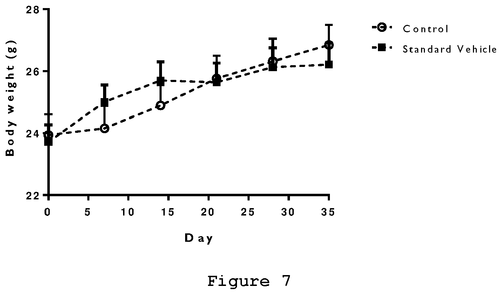

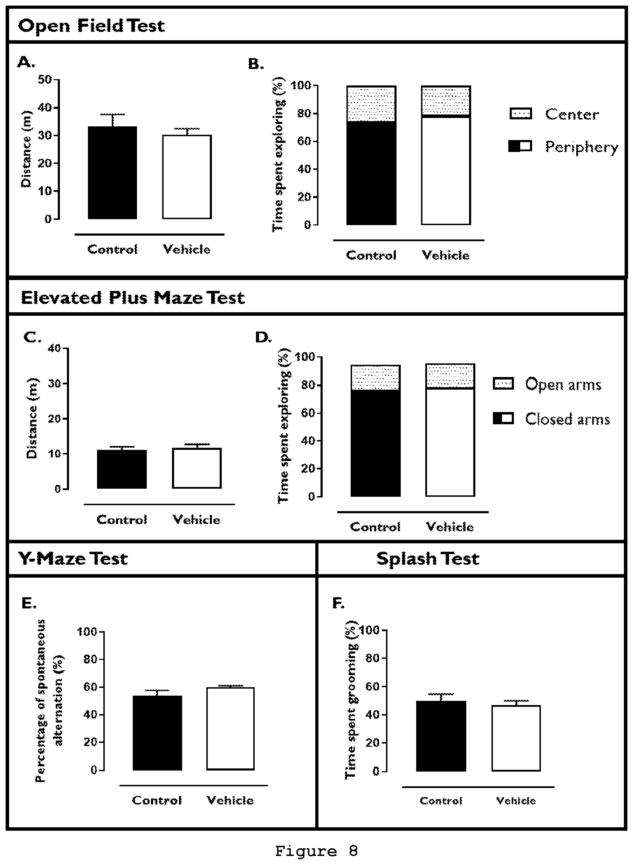

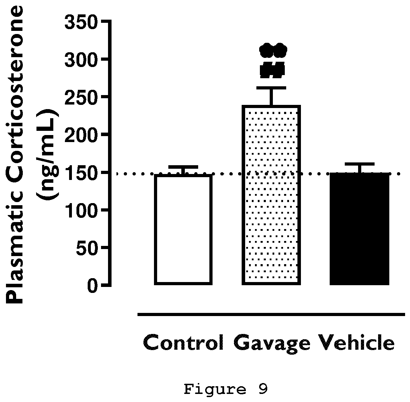

Since stress may impact nearly all the body's homeostatic mechanisms including body weight control and food/water consumption, clinical signs of animal well-being need to be carefully monitored throughout the entire preclinical experimentation protocol. It is demonstrated herein strong experimental evidence regarding rodents stress-free phenotype following a chronic semi-solid pill regimen, namely: i) regular food/water consumption and normal growth rate curve (Fig 7); ii) normal exploratory/locomotion behavioral phenotype in a battery of stress-related behavioral tests (Fig 8) and iii) lack of stress-related typical readouts such as disturbed pelage/ruffled fur, abnormal gait or posture or porphyrin rings around the eyes. Furthermore, semi-solid pill administration circumvents the rise of plasmatic corticosterone levels (a stress-related hormone) typically observed following gavage protocol (Fig. 9), corroborating the stress-free nature of present invention.

Additionally, the present invention allows the preservation of several physiological parameters that are paramount for preclinical assays within drug discovery process. Chronic semi-solid pill administration maintains normal ileum motility (Fig 10), an important aspect for oral drug pharmacokinetics studies. Moreover, it preserves serum glucose/lipidic profile, liver and kidney histology / function, hinting for metabolic homeostasis (Fig 11, 12). Altogether, these features endure semi-solid pill's safety.

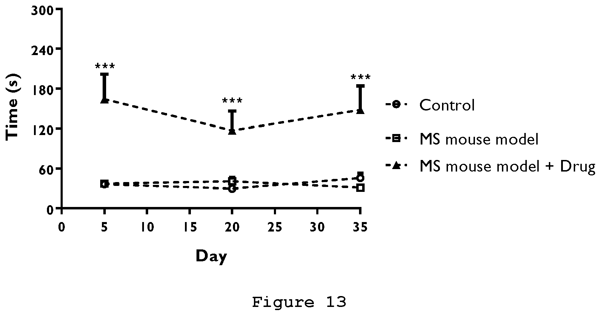

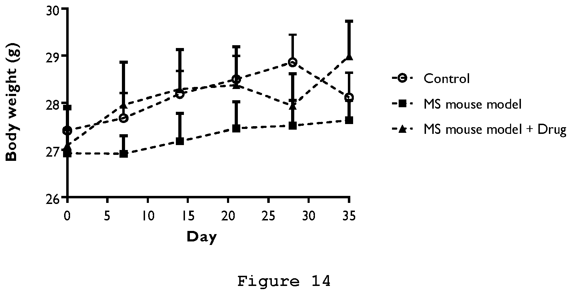

To further assess the suitability of the semi-solid pill formulation in diseased experimental models, a semi-solid pill chronic administration was performed in a cuprizone (CPZ) intoxication (0,2% in drinking water) setting for 35 days (5 weeks), a well-validated model of demyelination mimicking several features of multiple sclerosis (Norkute et al. 2009; Yamamoto et al. 2014) . This model was chosen taking into account the cognitive and motor deficits that could severely compromise the pre-training learning phase, motor skills and long-lasting acceptance (Austen, Strickland, and Sanderson 2016). Data depicted in Figure 13 clearly shows that CPZ-intoxicated subjects (MS animal model group) exhibit a "voluntary acceptance index" similar to control group. Moreover, chronic semi-solid-pill administration also provides a similar pattern of body weight evolution between healthy- and diseased experimental subjects (Fig 14), reinforcing the potential applicability in pre-clinical assays of drug development.

To evaluate rodents "voluntary acceptance index" efficacy of this new vehicle carrying a pharmacological agent, the antidiabetic drug sitagliptin - a dipeptidyl peptidase (DPPIV) IV inhibitor - was selected regarding their anorexigenic and nausea-induced properties which are challenging side-effects for chronic consumption of semi-solid pills (Webb et al. 2017). Sitagliptin (50mg/Kg body weight) was incorporated into semi-solid pills and daily administered into a third batch of CPZ-intoxicated animals (n=8). The time spent on voluntary consumption was less than 3 minutes (Fig 13), also within the preferred "voluntary acceptance index" (Thombre & Kasraian 2003).

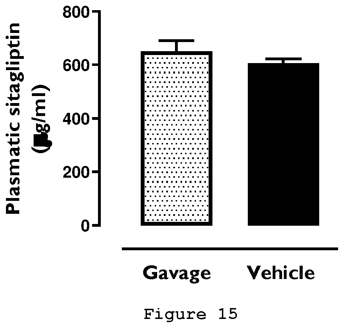

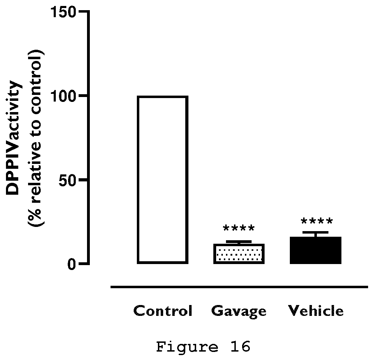

To provide undoubtful evidence of semi-solid pill efficacy regarding drug delivery/action, plasmatic stigaliptin concentration was assessed 2 hours post-dosing (50mg/Kg body weight) and compared with gavage administration protocol. Remarkably, similar results were obtained when both methodologies were used, proving the efficiency of this new invention in bioactive compounds delivery (Fig. 15). In addition, this new formula is unequivocally effective in delivering bioactive compounds into rodents' bloodstream and subsequent pharmacological effect, taking into account the plasmatic sitagliptin concentration data and the robust decrease (≈ 80-90%) on DPPIV activity (Fig 16).

The semi-solid pill methodology proposed herein is reproducible, with similar results obtained for additional 5 batches of C57BL/6 males (n=8/each) and 2 batches of C57BL/6 females (n=8/each), in a total of 56 experimental subjects. In two batches of C57BL/6 males, the voluntary acceptance remained constant until day 50, suggesting that this formulation may be administered for longer periods of chronic bioactive compounds treatments.

The data gathered in the exemplary studies presented herein and described in the Examples and Figures indicate that the invention can provide a valuable method for preclinical pharmacological and toxicological assays in experimental rodents. Furthermore, the present invention provides a new alternative for domestic toxicants baits.

Experiments conducted in support of this invention were performed with C57BL/6 mice and Wistar rats that are commonly used for biomedical studies. For instance, C57BL/6 mice display a close genetic relationship to humans and its genome is easily modified to study human disease. In fact, due to the availability of congenic strains, easy breeding, robustness and reproducibility, C57BL/6 mice is by far the most popular laboratory rodent. Animal accommodation and experimentation was performed in the animal facility of Coimbra Institute for Clinical and Biomedical Research from the Faculty of Medicine of University of Coimbra, under controlled environment conditions [12-h light/dark schedule at room temperature (2311 °C), with food and water ad libitum] and in strict accordance with European Community directive (2010/63/EU).

It therefore is disclosed herein a semi-solid pill formulation for the voluntary oral administration of bioactive compounds to rodents, wherein said formulation comprises a non-digestible sweetener, a thickening agent, a diluent agent, flavor masking agent and the bioactive compound to be administered. The formulation may further comprise preservatives.

In the context of the present patent application, a "non-digestible sweetener" or "non-digestible sugar" is understood as a rodent suitable sweetener that is not absorbed by the animal's digestive system, thus not affecting its metabolism. Suitable examples of non-digestible sweeteners are calcium saccharin and sucralose.

In the context of the present patent application, a "thickening agent" or "gellant agent" is understood as a rodent suitable substance which increases the viscosity of the formulation without interacting or altering the properties of the bioactive compound to be administered. Suitable examples of thickening agents are gelatin, agar, carrageenan, sodium alginate and pectin.

In the context of the present patent application, a "diluent agent" is understood as an inert and non-toxic powder, physically and chemically stable by itself as well as in combination with bioactive compounds, that act as a filler improving both pill content uniformity/cohesion as well as rodent's voluntary acceptance due to pleasant organoleptic properties (e.g. unsticky cool effect, taste enhancer). Suitable examples of diluent agents are powders with a granulometry between 300 an 800pm obtained from commercially available seeds, cereal grains or cereal grain based standard diets for rodents. Typical compositions rely on de-hulled extracted toasted soya, soya oil, wheat, barley, wheatfeed, soya protein concentrate, whey powder, aminoacids, vitamins, microminerals, macrominerals, among others.

In the context of the present patent application, a "bioactive compound" is understood as a chemical substance (or a mixture of chemical substances), usually derived from i) a natural source (living organisms such as plants, animals and/or microorganisms),ii) chemical synthesis or ii) biotechnological sources, which is pharmacologically active [e.g. active pharmaceutical ingredients (APIs), toxins, vegetable biomasses] when administered through the oral route.

**Table 1. Semi-solid pill formula**

| **FORMULA** | **PERCENTAGE** |
|---|---|
| **i) Non-digestible sweetener** | |
| **Examples (one of the following)** : | |
| **• Calcium saccharin** | 0.1-0.25% m/v |
| **• Sucralose** | 1-5% m/v |
| **ii) Thickening agent** | |
| **Examples(one of the following):** | |
| **• Gelatin (vegetable or animal source)** | 5-20% m/v |
| **• Agar** | 1-5% m/v |
| **• Carrageenan** | 0.02-3% m/v |
| **• Sodium alginate** | 0.7-2% m/v |
| **• Pectin** | 0.5-5% |
| **iii) Diluent agent** | |
| **Examples (one of the following) :** | 15-30 % m/v |
| **• Seeds (e.g. sunflower, canary)** | 15-30 % m/v |
| • Cereal grain or cereal grain based s**tandard diet for rodents** | |
| **(Preferred granulometry 300-800um)** | |
| **iv) Taste masking agent** | |
| **• Flavoring essence** | (as needed) |
| **v) Preservatives/Stabilizers** | (as needed) |
| **vi) Water** | (as needed for the desired final volume) |
| **vii) Bioactive compounds** | (as needed) |

**Table 2. Semi-solid pill nutritional composition (Standard vehicle composition: gelatin porcine skin 10% m/v, sucralose 2%, strawberry flavoring essence 8% v/v, cereal grain based standard diet for rodents 15%; Low-protein vehicle composition: Agar 2% m/v, sucralose 2%, strawberry flavoring essence 8% v/v, cereal grain based standard diet for rodents 15%) .**

| | **Semi-solid pill** | **g/Semi-solid pill** | **Kcal/Semi-solid pill** |
|---|---|---|---|
| **STANDARD FORMULA** | **Protein** | 0.0202 | 0.0807 |
| | **Fat** | 0.0004 | 0.0041 |
| | **Carbohydrates** | 0.0080 | 0.0320 |
| | **Total calories** | - | 0.1169 |
| **LOW PROTEIN FORMULA** | **Protein** | 0.0029 | 0.0116 |
| | **Fat** | 0.0004 | 0.0040 |
| | **Carbohydrates** | 0.0095 | 0.0381 |
| | **Total calories** | - | 0.0538 |

### EXAMPLES

The invention will now be illustrated in the following non-limiting examples which are illustrative of the invention but are not intended to limit the scope of the invention.

### Example 1

A mixture of agar (1,5% m/v), vegetable origin gelatin (5% m/v) and calcium saccharin (0,15% m/v) solution are immersed in a water bath (35-40°C). Apple flavoring essence (1% v/v), sunflower seeds (20% m/v), fumaric acid (2.5% m/v), sodium citrate (1.2% m/v) and methyl/propyl paraben (2:1, 0.05% v/v) are added to mixture and stirred 5 minutes for proper mixing. Rosuvastatin 5mg/Kg body weight) is transferred into each well of a 24/96-well plate round bottom (with lid) and admixed with the desired volume of vehicle. Plate is covered with a lid and semi-solid pill acquire the semi-solid texture within 30 minutes at 4°C.

### Example 2

A mixture of agar (2% m/v) and sucralose (1% m/v) solution are immersed in a water bath (35-40°C). Vanilla flavoring essence (1% v/v), cereal grain based standard diet for rodents (20% m/v), fumaric acid (2.5% m/v), sodium citrate (1.2% m/v) and methyl/propyl paraben (2:1, 0.05% v/v) are added to mixture and stirred 5 minutes for proper mixing. Atorvastatin 5mg/Kg body weight) is transferred into each well of a 24/96-well plate round bottom (with lid) and admixed with the desired volume of vehicle. Plate is covered with a lid and semi-solid pill acquire the semi-solid texture within 10 minutes at 4°C. Wherein the cereal grain based standard diet for rodents comprised dehulled soybean meal, wheat middlings, flaked corn, ground corn, fish meal, cane molasses, ground wheat, dried whey, soybean oil and brewers dried yeast.

### Example 3

A mixture of pectin (1% m/v), agar (1% m/v) and sucralose (1,5% m/v) solution are immersed in a water bath (35-40°C). Blueberry flavoring essence (1% v/v), canary seeds (30% m/v), fumaric acid (2,5% m/v), sodium citrate (1,2% m/v) and methyl/propyl paraben (2:1, 0.05% v/v) are added to mixture and stirred 5 minutes for proper mixing. Bromadiolone (0.5 mg/Kg body weight) is transferred into each well of a 24/96-well plate round bottom (with lid) and admixed with the desired volume of vehicle. Plate is covered with a lid and semi-solid pill acquire the semi-solid texture within 30 minutes at 4°C.

### Example 4

A mixture of gelatin porcine skin (7,5% m/v), pectin (1% m/v) and sucralose (2% m/v) solution are immersed in a water bath (35-40°C). Strawberry flavoring essence (8% v/v), cereal grain based standard diet for rodents (20% m/v), fumaric acid (2,5% m/v), sodium citrate (1,2% m/v) and methyl/propyl paraben (2:1, 0.05% v/v) are added to mixture and stirred 5 minutes for proper mixing. Sitagliptin (50mg/Kg body weight) is transferred into each well of a 24/96-well plate round bottom (with lid) and admixed with the desired volume of vehicle. Plate is covered with a lid and semi-solid pill acquire the semi-solid texture within 30 minutes at 4°C. Wherein the cereal grain based standard diet for rodents comprised dehulled soybean meal, wheat middlings, flaked corn, ground corn, fish meal, cane molasses, ground wheat, dried whey, soybean oil and brewers dried yeast.

### Example 5

A mixture of vegetable origin gelatin (20% m/v) and sucralose (1% m/v) solution are immersed in a water bath (35-40°C). Berry flavoring essence (1% v/v), cereal grain based standard diet for rodents (20% m/v), fumaric acid (2.5% m/v), sodium citrate (1.2% m/v) and methyl/propyl paraben (2:1, 0.05% v/v) are added to mixture and stirred 5 minutes for proper mixing. Fluoxetin 2.6 mg/Kg body weight) is transferred into each well of a 24/96-well plate round bottom (with lid) and admixed with the desired volume of vehicle. Plate is covered with a lid and semi-solid pill acquire the semi-solid texture within 30 minutes at 4°C. Wherein the cereal grain based standard diet for rodents comprised dehulled soybean meal, wheat middlings, flaked corn, ground corn, fish meal, cane molasses, ground wheat, dried whey, soybean oil and brewers dried yeast.

### Example 6

A mixture of gelatin bovine skin (10% m/v), agar (1% m/v), and sucralose (1% m/v) solution are immersed in a water bath (35-40°C). Blueberry flavoring essence (1% v/v), sunflower seeds (20% m/v), fumaric acid (2.5% m/v), sodium citrate (1.2% m/v) and methyl/propyl paraben (2:1, 0.05% v/v) are added to mixture and stirred 5 minutes for proper mixing. Prednisolone 10mg/Kg body weight) is transferred into each well of a 24/96-well plate round bottom (with lid) and admixed with the desired volume of vehicle. Plate is covered with a lid and semi-solid pill acquire the semi-solid texture within 30 minutes at 4°C.

### Example 7

A mixture of carrageenan (0,1% m/v) and calcium saccharin (0,15% m/v) solution are immersed in a water bath (35-40°C). Berry flavoring essence (1% v/v), cereal grain based standard diet for rodents (30% m/v), fumaric acid (2,5% m/v), sodium citrate (1,2% m/v) and methyl/propyl paraben (2:1, 0.05% v/v) are added to mixture and stirred 5 minutes for proper mixing. Blueberry leaves biomass (1.6 g/Kg body weight) is transferred into each well of a 24/96-well plate round bottom (with lid) and admixed with the desired volume of vehicle. Plate is covered with a lid and semi-solid pill acquire the semi-solid texture within 30 minutes at 4°C. Wherein the cereal grain based standard diet for rodents comprised dehulled soybean meal, wheat middlings, flaked corn, ground corn, fish meal, cane molasses, ground wheat, dried whey, soybean oil and brewers dried yeast.

### Example 8

A mixture of sodium alginate (1% m/v) and sucralose (1,5% m/v) solution are immersed in a water bath (35-40°C). Strawberry flavoring essence (8% v/v), cereal grain based standard diet for rodents (30% m/v), fumaric acid (2,5% m/v), sodium citrate (1,2% m/v) and methyl/propyl paraben (2:1, 0.05% v/v) are added to mixture and stirred 5 minutes for proper mixing. Sertraline (5 mg/Kg body weight) is transferred into each well of a 24/96-well plate round bottom (with lid) and admixed with the desired volume of vehicle. Plate is covered with a lid and semi-solid pill acquire the semi-solid texture within 30 minutes at 4°C. Wherein the cereal grain based standard diet for rodents comprised dehulled soybean meal, wheat middlings, flaked corn, ground corn, fish meal, cane molasses, ground wheat, dried whey, soybean oil and brewers dried yeast.

### Example 9

A mixture of gelatin bovine skin (10% m/v), sodium alginate (1% m/v) and sucralose (1% m/v) solution are immersed in a water bath (35-40°C). Vanilla flavoring essence (1% v/v), sunflower seeds (20% m/v), fumaric acid (2.5% m/v), sodium citrate (1.2% m/v) and methyl/propyl paraben (2:1, 0.05% v/v) are added to mixture and stirred 5 minutes for proper mixing. Warfarin (80mg/Kg body weight) is transferred into each well of a 24/96-well plate round bottom (with lid) and admixed with the desired volume of vehicle. Plate is covered with a lid and semi-solid pill acquire the semi-solid texture within 30 minutes at 4°C.

### Example 10

A mixture of gelatin bovine skin (20% m/v) and sucralose (1% m/v) solution are immersed in a water bath (35-40°C) . Vanilla flavoring essence (1% v/v), canary seeds (20% m/v), fumaric acid (2.5% m/v), sodium citrate (1.2% m/v) and methyl/propyl paraben (2:1, 0.05% v/v) are added to mixture and stirred 5 minutes for proper mixing. Metformin 100mg/Kg body weight) is transferred into each well of a 24/96-well plate round bottom (with lid) and admixed with the desired volume of vehicle. Plate is covered with a lid and semi-solid pill acquire the semi-solid texture within 30 minutes at 4°C.

### Example 11

A mixture of gelatin bovine skin (20% m/v) and sucralose (1% m/v) solution are immersed in a water bath (35-40°C) . Vanilla flavoring essence (1% v/v), cereal grain based standard diet for rodents (20% m/v), fumaric acid (2.5% m/v), sodium citrate (1.2% m/v) and methyl/propyl paraben (2:1, 0.05% v/v) are added to mixture and stirred 5 minutes for proper mixing. Capsaicin 0,1mg/Kg body weight) is transferred into each well of a 24/96-well plate round bottom (with lid) and admixed with the desired volume of vehicle. Plate is covered with a lid and semi-solid pill acquire the semi-solid texture within 30 minutes at 4°C. Wherein the cereal grain based standard diet for rodents comprised dehulled soybean meal, wheat middlings, flaked corn, ground corn, fish meal, cane molasses, ground wheat, dried whey, soybean oil and brewers dried yeast.

Several features are described hereafter that can each be used independently of one another or with any combination of the other features. However, any individual feature might not address any of the problems discussed above or might only address one of the problems discussed above. Some of the problems discussed above might not be fully addressed by any of the features described herein. Although headings are provided, information related to a particular heading, but not found in the section having that heading, may also be found elsewhere in the specification.

### Brief description of drawings

For easier understanding of this application, figures are attached in the annex that represent the preferred forms of implementation which nevertheless are not intended to limit the technique disclosed herein.
Figure 1 shows voluntary acceptance index of prior art formula (Zhang et al.) versus present invention formula (Vehicle composition: gelatin porcine skin 10% m/v, sucralose 2%, strawberry flavoring essence 8% v/v, cereal grain based standard diet for rodents 15%). Data are presented as mean ± S.E.M. (n= 6-8 experimental group).
Figure 2 shows Semi-solid vehicle turbidity over time. The transmitted light, assessed through spectrophotometry, was used as a readout of chemical stability under RT or 4°C storage. Standard vehicle composition: gelatin porcine skin 20% m/v, sucralose 2%, strawberry flavoring essence 8% v/v, cereal grain based standard diet for rodents 15% and preservatives [tartaric acid (E334) and sodium citrate (E331) added according to EMA dosage recommendations].
Figure 3 illustrates the semi-solid pill administration setup.
Figure 4 shows the semi-solid pill vehicle acceptance over time in healthy rodents (A: C57BL/6 mice, B: Wistar rats). Standard vehicle composition: gelatin porcine skin 10% m/v, sucralose 2%, strawberry flavoring essence 8% v/v, cereal grain based standard diet for rodents 15%; Low-protein vehicle composition: Agar 2% m/v, sucralose 2%, strawberry flavoring essence 8% v/v, standard diet for rodents 15%). Data are presented as mean ± S.E.M. (n= 6-8 experimental group) .
Figure 5 shows the semi-solid pill vehicle acceptance carrying bioactive compounds over time in healthy C57BL/6 mice (Vehicle composition: described in Examples, Section 3.3). Data are presented as mean ± S.E.M. (n= 6-8 experimental group).
Figure 6 shows the semi-solid pill vehicle acceptance carrying Warfarin (80mg/Kg body weight), a rodenticide in healthy C57BL/6 mice (Vehicle composition: described in Examples, Section 3.3). Data are presented as mean ± S.E.M. (n= 6-8 experimental group).
Figure 7 shows body weight evolution after chronic vehicle semi-solid pill administration (Vehicle composition: gelatin porcine skin 10% m/v, sucralose 2%, strawberry flavoring essence 8% v/v, cereal grain based standard diet for rodents 15%). Data are presented as mean ± S.E.M. (n= 6-8 experimental group).
Figure 8 shows behavioral tests to ascertain stress-related parameters after chronic vehicle semi-solid pill administration (35 consecutive days). Open field for exploration habits, Elevated Plus Maze for anxiety-like parameters, Y-Maze for willingness to explore new environments and Splash test for self-care and motivation. (Vehicle composition: gelatin porcine skin 10% m/v, sucralose 2%, strawberry flavoring essence 8% v/v, cereal grain based standard diet for rodents 15%) . Data are presented as mean ± S.E.M. (n= 11-12 experimental group).
Figure 9 shows corticosterone plasmatic concentration 30 minutes following a single vehicle administration through forced gavage versus voluntary semi-solid vehicle consumption (Vehicle composition: gelatin porcine skin 10% m/v, sucralose 2%, strawberry flavoring essence 8% v/v, standard diet for rodents 15%). Data are presented as mean ± S.E.M. (n= 5-7 experimental group. One-way ANOVA, ** P<0.01 versus control, ## P<0.01 versus vehicle).

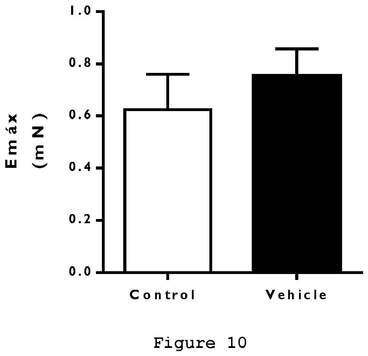

Figure 10 shows Ileum contractility after chronic vehicle semi-solid pill administration (35 consecutive days. Vehicle composition: gelatin porcine skin 10% m/v, sucralose 2%, strawberry flavoring essence 8% v/v, cereal grain based standard diet for rodents 15%). Data are presented as mean ± S.E.M. (n= 6-8 experimental group).

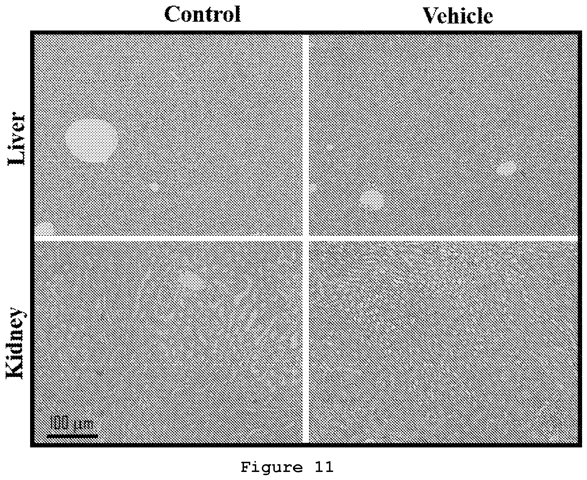

Figure 11 illustrates Hematoxylin & eosin staining (10x magnification) of liver and kidney sections after chronic vehicle semi-solid pill administration ((35 consecutive days. Vehicle composition: gelatin porcine skin 10% m/v, sucralose 2%, strawberry flavoring essence 8% v/v, cereal grain based standard diet for rodents 15%).

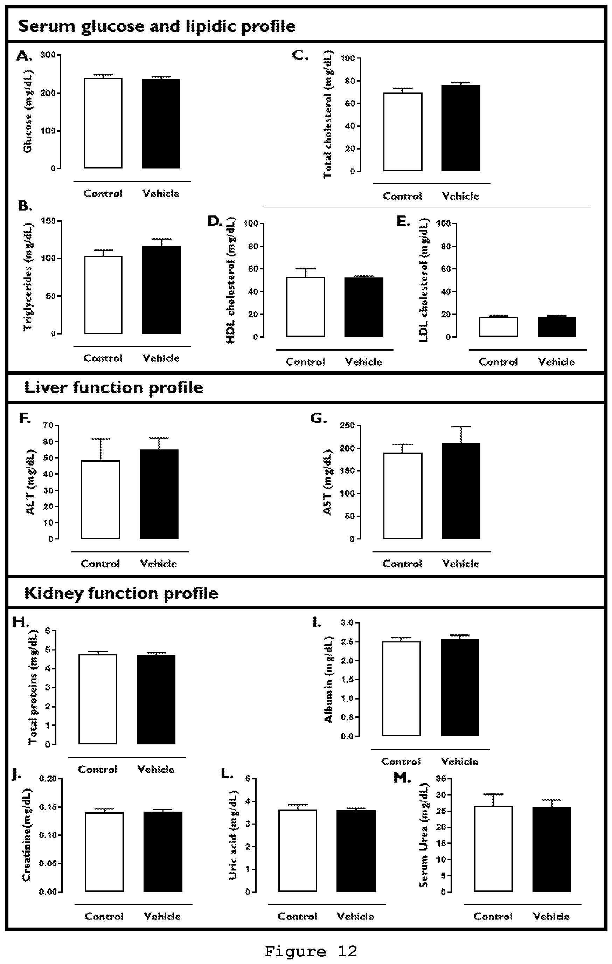

Figure 12 shows metabolic profile and biochemical markers of liver and kidney function after chronic vehicle administration (35 consecutive days). HDL, high-density lipoprotein; LDL, low-density lipoprotein; ALT, alanine transaminase; AST, aspartate transaminase. (Vehicle composition: gelatin porcine skin 10% m/v, sucralose 2%, strawberry flavoring essence 8% v/v, cereal grain based standard diet for rodents 15%). Data are presented as mean ± S.E.M. (n= 6-8 experimental group).
Figure 13 shows semi-solid pill vehicle acceptance over time in an experimental mouse model of multiple sclerosis (Vehicle composition: gelatin porcine skin 10% m/v, sucralose 2%, strawberry flavoring essence 8% v/v, cereal grain based standard diet for rodents 15%). Data are presented as mean ± S.E.M. (One-way ANOVA, *** P<0.001 versus control, n= 6-8 experimental group).
Figure 14 shows Body weight evolution after chronic semi-solid pill administration in an experimental mouse model of multiple sclerosis (Vehicle composition: gelatin porcine skin 10% m/v, sucralose 2%, strawberry flavoring essence 8% v/v, cereal grain based standard diet for rodents 15%). Data are presented as mean ± S.E.M.
Figure 15 illustrates plasmatic sitagliptin concentration (HPLC/UV) following sitagliptin administration (50mg/Kg, once) through gavage or voluntary semi-solid vehicle consumption Data are presented as mean ± S.E.M. (n= 5-6 experimental group). (Vehicle composition: gelatin porcine skin 10% m/v, sucralose 2%, strawberry flavoring essence 8% v/v, cereal grain based standard diet for rodents 15%).
Figure 16 shows *In vivo* drug efficacy after sitagliptin administration (50mg/Kg, once) through gavage or through semi-solid pill . Plasmatic DPPIV activity was measured to assess sitagliptin pharmacological action (Vehicle composition: gelatin porcine skin 2% m/v, sucralose 2%, strawberry flavoring essence 8% v/v, cereal grain based standard diet for rodents 15%). Data are presented as mean ± S.E.M. (One-way ANOVA, **** P<0.0001 versus control, n= 6-8 experimental group).

### Description of the embodiments

Now, preferred embodiments of the present application will be described in detail. However, they are not intended to limit the scope of this application.

It is disclosed herein a semi-solid pill formulation for the voluntary oral administration of bioactive compounds to rodents, wherein said formulation comprises:
- at least one thickening agent, wherein said thickening agent is selected from gelatin, agar, carrageenan, sodium alginate and pectin;
- a digestible sweetener, wherein the digestible sweetener is selected from sucralose and calcium saccharin;
- a diluent agent, wherein the diluent agent is a powder with a granulometry between 300 and 800pm,obtained from finely grounded seeds , cereal grains or cereal grain based diet for rodents, in a concentration between 10 and 30% m/v;
- at least one flavor masking agent;
- a bioactive compound.

In one embodiment, the finely grounded seeds are selected from at least one of sunflower and canary.

In one embodiment, the finely grounded cereal grain based diet for rodents comprise at least one of dehulled soybean meal, wheat middlings, flaked corn, ground corn, fish meal, cane molasses, ground wheat, dried whey, soybean oil and brewers dried yeast.

In one embodiment, the formulation further comprises a preservative.

In one embodiment, the thickening agent is gelatin in a concentration between 5 and 20% m/v.

In another embodiment, the thickening agent is agar in a concentration between 1 and 5% m/v.

In another embodiment, the thickening agent is carrageenan in a concentration between 0.02 and 3% m/v.

In another embodiment, the thickening agent is sodium alginate in a concentration between 0.7 and 2% m/v.

In another embodiment, the thickening agent is pectin in a concentration between 0.5 and 5% m/v.

In one embodiment, the non-digestible sweetener is sucralose in a concentration between 1 and 5% m/v.

In another embodiment, the non-digestible sweetener is calcium saccharin in a concentration between 0.1 and 0.25% m/v.

In one embodiment, the flavor masking agent is selected from vanilla, strawberry, blueberry and chocolate.

In one embodiment, the formulation of the present patent application is for use in the oral administration of drugs and bioactive compounds to rodents.

In another embodiment, the formulation of the present patent application is used in toxicological screening methods of rodents.

In another embodiment, the formulation of the present patent application is used in the production of toxicant baits for rodents.

### REFERENCES

Abelson, Klas S P, Kirsten R. Jacobsen, Renée Sundbom, Otto Kalliokoski, and Jann Hau. 2012. "Voluntary Ingestion of Nut Paste for Administration of Buprenorphine in Rats and Mice." Laboratory Animals. https://doi.org/10.1258/la.2012.012028.
Atcha, Zeenat, Claire Rourke, Aveline Hp Neo, Catherine Wh Goh, Jean Sk Lim, Chiu-Cheong Aw, Edward R Browne, and Darrel J Pemberton. 2010. "Alternative Method of Oral Dosing for Rats." J Am Assoc Lab Anim Sci.
Austen, Joseph M., Jasmin A. Strickland, and David J. Sanderson. 2016. "Memory-Dependent Effects on Palatability in Mice." Physiology and Behavior. https://doi.org/10.1016/j.physbeh.2016.09.001.
Bouet V, Boulouard M, Toutain J, Divoux D, Bernaudin M, Schumann-Bard P, Freret T. 2009 "The adhesive removal test: a sensitive method to assess sensorimotor deficits in mice". Nat Protoc. 2009;4(10):1560-4. doi: 10.1038/nprot.2009.125. Brown, A P, N Dinger, and B S Levine. 2000. "stress Produced by Gavage Administration in the Rat." Contemporary Topics American Association Fo Laboratory Animal Science. https://doi.org/10.1136/ejhpharm-2015-000639.216.
Chattopadhyay, Sanchari, Utpal Raychaudhuri, and Runu Chakraborty. 2014. "Artificial Sweeteners - A Review." Journal of Food Science and Technology. https://doi.org/10.1007/s13197-011-0571-1.
Dhawan, Sandeep S., Xia Shuang, Tait, David S., Bundgaard, Christoffer, Bowman Ellen and Brown Verity J. 2018. "Oral dosing of rodents using a palatable tablet" Psychopharmacology (Berl) doi: 10.1007/s00213-018-4863-2.
Diogo, Lucilia N, Inês V Faustino, Ricardo A Afonso, Sofia A Pereira, Emilia C Monteiro, and Ana I Santos. 2015. "Voluntary Oral Administration of Losartan in Rats." Journal of the American Association for Laboratory Animal Science: JAALAS. https://doi.org/10.14288/1.0076108.
Fontaine, Danielle A., and Dawn Belt Davis. 2016. "Attention to Background Strain Is Essential for Metabolic Research: C57BL/6 and the International Knockout Mouse Consortium." Diabetes. https://doi.org/10.2337/db15-0982.
Gonzales, C., M. M. Zaleska, D. R. Riddell, K. P. Atchison, A. Robshaw, H. Zhou, and S. J. Sukoff Rizzo. 2014. "Alternative Method of Oral Administration by Peanut Butter Pellet Formulation Results in Target Engagement of BACE1 and Attenuation of Gavage-Induced Stress Responses in Mice." Pharmacology Biochemistry and Behavior. https://doi.org/10.1016/j.pbb.2014.08.010.
Huang-Brown, K M, and F A Guhad. 2002. "Chocolate, an Effective Means of Oral Drug Delivery in Rats." Lab Anim (NY). https://doi.org/10.1038/5000197.
Huynh, Nhat, Natalie Arabian, Dustin Lieu, Liana Asatryan, and Daryl L Davies. 2016. "Utilizing an Orally Dissolving Strip for Pharmacological and Toxicological Studies: A Simple and Humane Alternative to Oral Gavage for Animals." Journal of Visualized Experiments: JoVE. https://doi.org/10.3791/53770.
Jones, Carissa P, Kelli L Boyd, and Jeanne M Wallace. 2016. "Evaluation of Mice Undergoing Serial Oral Gavage While Awake or Anesthetized." Journal of the American Association for Laboratory Animal Science.
Kopf, Phillip G., Jason A. Scott, Larry N. Agbor, Jason R. Boberg, Khalid M. Elased, Janice K. Huwe, and Mary K. Walker. 2010. "Cytochrome P4501A1 Is Required for Vascular Dysfunction and Hypertension Induced by 2,3,7,8-Tetrachlorodibenzo-p-Dioxin." Toxicological Sciences. https://doi.org/10.1093/toxsci/kfq218.
Küster, Tatiana, Beatrice Zumkehr, Corina Hermann, Regula Theurillat, Wolfgang Thormann, Bruno Gottstein, and Andrew Hemphill. 2012. "Voluntary Ingestion of Antiparasitic Drugs Emulsified in Honey Represents an Alternative to Gavage in Mice." J Am Assoc Lab Anim Sci.
Liles, J. H., P. A. Flecknell, J. Roughan, and I. Cruz-Madorran. 1998. "Influence of Oral Buprenorphine, Oral Naltrexone or Morphine on the Effects of Laparotomy in the Rat." Laboratory Animals. https://doi.org/10.1258/002367798780600025.
Lim GB. 2017 "Milestone 2: Warfarin: from rat poison to clinical use." Nat Rev Cardiol. doi: 10.1038/nrcardio.2017.172. PubMed PMID: 23930179
Lund, M. 1988: Selection of baits and their distribution. Pp. 261-268 in Prakash, I. (Ed.) : Rodent pest management. CRC Press, Boca Raton, Florida, US ISBN:9781351093354 Molina-Cimadevila, Maria Jesus, S. Segura, C. Merino, N. Ruiz-Reig, B. Andrés, and E. De Madaria. 2014. "Oral Self-Administration of Buprenorphine in the Diet for Analgesia in Mice." Laboratory Animals. https://doi.org/10.1177/0023677214532454.
Norkute, Akvile, Andrea Hieble, Alena Braun, Sonja Johann, Tim Clarner, Werner Baumgartner, Cordian Beyer, and Markus Kipp. 2009. "Cuprizone Treatment Induces Demyelination and Astrocytosis in the Mouse Hippocampus." Journal of Neuroscience Research. https://doi.org/10.1002/jnr.21946.
Rivera, Juan, and Lino Tessarollo. 2008. "Genetic Background and the Dilemma of Translating Mouse Studies to Humans." Immunity. https://doi.org/10.1016/j.immuni.2007.12.008.
Schleimer, Sonja B., Graham A.R. Johnston, and Jasmine M. Henderson. 2005. "Novel Oral Drug Administration in an Animal Model of Neuroleptic Therapy." Journal of Neuroscience Methods. https://doi.org/10.1016/j.jneumeth.2005.02.004.
Sengupta P. 2013 "The Laboratory Rat: Relating Its Age With Human's." Int J Prev Med.
2013 Jun;4(6):624-30.; PubMed Central PMCID: PMC3733029. Sobolewski, Marissa, Joshua L. Allen, Keith Morris-Schaffer, Carolyn Klocke, Katherine Conrad, and Deborah A. Cory-Slechta. 2016. "A Novel, Ecologically Relevant, Highly Preferred, and Non-Invasive Means of Oral Substance Administration for Rodents." Neurotoxicology and Teratology. https://doi.org/10.1016/j.ntt.2016.04.002.
Stewart, Katie D., Joseph A. Johnston, Louis S. Matza, Sarah E. Curtis, Henry A. Havel, Stephanie A. Sweetana, and Heather L. Gelhorn. 2016. "Preference for Pharmaceutical Formulation and Treatment Process Attributes." Patient Preference and Adherence. https://doi.org/10.2147/PPA.S101821.
Taylor, Bryan F, Harvey E Ramirez, August H Battles, Karl A Andrutis, and John K Neubert. 2016. "Analgesic Activity of Tramadol and Buprenorphine after Voluntary Ingestion by Rats (Rattus Norvegicus)." Journal of the American Association for Laboratory Animal Science: JAALAS.
Tordoff, Michael G., and Alexander A. Bachmanov. 2003. "Mouse Taste Preference Tests: Why Only Two Bottles?" Chemical Senses. https://doi.org/10.1093/chemse/28.4.315.
Turner, P. V., T. Brabb, C. Pekow, and M. A. Vasbinder. 2011. "Administration of Substances to Laboratory Animals: Routes of Administration and Factors to Consider." J Am Assoc Lab Anim Sci. https://doi.org/10.1016/j.jad.2013.05.099.
Vandenberg, Laura N., Wade V. Welshons, Frederick S.Vom Saal, Pierre Louis Toutain, and John Peterson Myers. 2014. "Should Oral Gavage Be Abandoned in Toxicity Testing of Endocrine Disruptors?" Environmental Health: A Global Access Science Source. https://doi.org/10.1186/1476-069X-13-46.
Viana, Sofia D., Inês R. Pita, Cristina Lemos, Daniel Rial, Patricia Couceiro, Paulo Rodrigues-Santos, Francisco Caramelo, et al. 2017. "The Effects of Physical Exercise on Nonmotor Symptoms and on Neuroimmune RAGE Network in Experimental Parkinsonism." Journal of Applied Physiology. https://doi.org/10.1152/japplphysiol.01120.2016.
Vidal-Martinez, Guadalupe, Javier Vargas-Medrano, Carolina Gil-Tommee, David Medina, Nathan T. Garza, Barbara Yang, Ismael Segura-Ulate, Samantha J. Dominguez, and Ruth G. Perez. 2016. "FTY720/Fingolimod Reduces Synucleinopathy and Improves Gut Motility in A53T Mice: Contributions of pro-Brain-Derived Neurotrophic Factor (PRO-BDNF) and Mature BDNF." Journal of Biological Chemistry. https://doi.org/10.1074/jbc.M116.744029.
Walker, Mary K., Jason R. Boberg, Mary T. Walsh, Valerie Wolf, Alisha Trujillo, Melissa Skelton Duke, Rupert Palme, and Linda A. Felton. 2012. "A Less Stressful Alternative to Oral Gavage for Pharmacological and Toxicological Studies in Mice." Toxicology and Applied Pharmacology. https://doi.org/10.1016/j.taap.2012.01.025.
Webb, Dominic Luc, Niclas Abrahamsson, Magnus Sundbom, and Per M. Hellström. 2017. "Bariatric Surgery-time to Replace with GLP-1?" Scandinavian Journal of Gastroenterology. https://doi.org/10.1080/00365521.2017.1293154.
Yamamoto, Shinji, Mari Gotoh, Yuuki Kawamura, Kota Yamashina, Sosuke Yagishita, Takeo Awaji, Motomu Tanaka, Kei Maruyama, Kimiko Murakami-Murofushi, and Keisuke Yoshikawa. 2014. "Cyclic Phosphatidic Acid Treatment Suppress Cuprizone-Induced Demyelination and Motor Dysfunction in Mice." European Journal of Pharmacology. https://doi.org/10.1016/j.ejphar.2014.07.040.
Yardley, Megan M., Nhat Huynh, Kathleen E. Rodgers, Ronald L. Alkana, and Daryl L. Davies. 2015. "Oral Delivery of Ivermectin Using a Fast Dissolving Oral Film: Implications for Repurposing Ivermectin as a Pharmacotherapy for Alcohol Use Disorder." Alcohol. https://doi.org/10.1016/j.alcohol.2015.03.006.
Zhang, Lei, and Lei Zhang. 2011. "Voluntary Oral Administration of Drugs in Mice." Protocol Exchange. https://doi.org/10.1038/protex.2011.236.

## Claims

1. A semi-solid pill formulation for the voluntary oral administration of bioactive compounds to rodents comprising:
- at least one thickening agent, wherein said thickening agent is selected from gelatin, agar, carrageenan, sodium alginate and pectin;
- a digestible sweetener, wherein the digestible sweetener is selected from sucralose and calcium saccharin;
- a diluent agent, wherein the diluent agent is a powder with a granulometry between 300 and 800µm in a concentration between 10 and 30% m/v, wherein the powder is selected from finely grounded seeds, cereal grains or finely grounded cereal grain based diet for rodents
- at least one flavor masking agent;
- a bioactive compound.

2. The semi-solid pill formulation according to claim 1, wherein the finely grounded seeds are selected from at least one of sunflower seeds and canary seeds.

3. The semi-solid pill formulation according to any of claims 1-2, wherein the finely grounded cereal grain based diet for rodents comprises at least one of dehulled soybean meal, wheat middlings, flaked corn, ground corn, fish meal, cane molasses, ground wheat, dried whey, soybean oil and brewers dried yeast.

4. The semi-solid pill formulation according to any of claims 1-3, wherein the semi-solid pill formulation further comprises a preservative.

5. The semi-solid pill formulation according to any of claims 1-4, wherein the thickening agent is gelatin in a concentration between 5 and 20% m/v.

6. The semi-solid pill formulation according to any of claims 1-5, wherein the thickening agent is agar in a concentration between 1 and 5% m/v.

7. The semi-solid pill formulation according to any of claims 1-6, wherein the thickening agent is carrageenan in a concentration between 0.02 and 3% m/v.

8. The semi-solid pill formulation according to any of claims 1-7, wherein the thickening agent is sodium alginate in a concentration between 0.7 and 2% m/v.

9. The semi-solid pill formulation according to any of claims 1-8, wherein the thickening agent is pectin in a concentration between 0.5 and 5% m/v.

10. The semi-solid pill formulation according to any of claims 1-9, wherein the non-digestible sweetener is sucralose in a concentration between 1 and 5% m/v.

11. The semi-solid pill formulation according to any of claims 1-10, wherein the non-digestible sweetener is calcium saccharin in a concentration between 0.1 and 0.25% m/v.

12. The semi-solid pill formulation according to any of claims 1-11, wherein the flavor masking agent is selected from vanilla, strawberry, blueberry and chocolate.

13. The semi-solid pill formulation described in claims 1-12, for use in the oral administration of drugs and bioactive compounds to rodents.

14. The semi-solid pill formulation described in claims 1-12, for use in toxicological screening methods of rodents.

15. The semi-solid pill formulation described in claims 1-12, for use in the production of toxicant baits for rodents.

## Patentansprüche

1. Eine halbfeste Pillenformulierung zur freiwilligen oralen Verabreichung von bioaktiven Verbindungen an Nagetiere, bestehend aus:
- Mindestens einem Verdickungsmittel, worin das genannte Verdickungsmittel aus Gelatine, Agar, Carrageen, Natriumalginat und Pektin ausgewählt wird;
- einem verdaulichen Süßstoff, worin der verdauliche Süßstoff aus Sucralose und Calciumsaccharin ausgewählt wird;
- einem Verdünnungsmittel, worin das Verdünnungsmittel ein Pulver mit einer Korngröße zwischen 300 und 800 pm bei einer Konzentration zwischen 10 und 30 % m/V ist, wobei das Pulver aus fein gemahlenen Samen, Getreidekörnern oder fein gemahlener getreidebasierter Nahrung für Nagetiere ausgewählt wird;
- mindestens einem Aroma-Maskierungsmittel;
- einer bioaktiven Verbindung.

2. Die halbfeste Pillenformulierung gemäß Anspruch 1, worin die fein gemahlenen Samen mindestens aus Sonnenblumenkernen und Kanariensamen ausgewählt werden.

3. Die halbfeste Pillenformulierung gemäß einem der Ansprüche 1-2, worin die fein gemahlene getreidebasierte Nahrung für Nagetiere mindestens aus geschältem Sojabohnenmehl, Weizenfuttermehl, Maisflocken, gemahlenem Mais, Fischmehl, Zuckerrohrmelasse, gemahlenem Weizen, getrockneter Molke, Sojabohnenöl und getrockneter Bierhefe besteht.

4. Die halbfeste Pillenformulierung gemäß einem der Ansprüche 1-3, worin die halbfeste Pillenformulierung weiterhin ein Konservierungsmittel umfasst.

5. Die halbfeste Pillenformulierung gemäß einem der Ansprüche 1-4, worin das Verdickungsmittel Gelatine mit einer Konzentration zwischen 5 und 20 % m/V ist.

6. Die halbfeste Pillenformulierung gemäß einem der Ansprüche 1-5, worin das Verdickungsmittel Agar mit einer Konzentration zwischen 1 und 5 % m/V ist.

7. Die halbfeste Pillenformulierung gemäß einem der Ansprüche 1-6, worin das Verdickungsmittel Carrageen mit einer Konzentration zwischen 0,02 und 3 % m/V ist.

8. Die halbfeste Pillenformulierung gemäß einem der Ansprüche 1-7, worin das Verdickungsmittel Natriumalginat mit einer Konzentration zwischen 0,7 und 2 % m/V ist.

9. Die halbfeste Pillenformulierung gemäß einem der Ansprüche 1-8, worin das Verdickungsmittel Pektin mit einer Konzentration zwischen 0,5 und 5 % m/V ist.

10. Die halbfeste Pillenformulierung gemäß einem der Ansprüche 1-9, worin der nicht verdauliche Süßstoff Sucralose mit einer Konzentration zwischen 1 und 5 % m/V ist.

11. Die halbfeste Pillenformulierung gemäß einem der Ansprüche 1-10, worin der nicht verdauliche Süßstoff Calciumsaccharin mit einer Konzentration zwischen 0,1 und 0,25 % m/V ist.

12. Die halbfeste Pillenformulierung gemäß einem der Ansprüche 1-11, worin das Aroma-Maskierungsmittel aus Vanille, Erdbeere, Heidelbeere und Schokolade ausgewählt wird.

13. Die halbfeste, in den Ansprüchen 1-12 beschriebene Pillenformulierung zur Verwendung bei der oralen Verabreichung von Medikamenten und bioaktiven Verbindungen an Nagetiere.

14. Die halbfeste, in den Ansprüchen 1-12 beschriebene Pillenformulierung zur Verwendung bei toxikologischen Screening-Verfahren von Nagetieren.

15. Die halbfeste, in den Ansprüchen 1-12 beschriebene Pillenformulierung zur Verwendung bei der Herstellung von giftigen Ködern für Nagetiere.

## Revendications

1. Formulation de pilule semi-solide pour l'administration orale volontaire de composés bioactifs à des rongeurs comprenant :
- au moins un agent épaississant, dans lequel ledit agent épaississant est sélectionné parmi la gélatine, l'agar, la carraghénane, l'alginate de sodium et la pectine ;
- un édulcorant digestible, dans lequel l'édulcorant digestible est sélectionné parmi le sucralose et la saccharine de calcium ;
- un agent diluant, dans lequel l'agent diluant est une poudre ayant une granulométrie comprise entre 300 et 800 pm en une concentration comprise entre 10 et 30 % m/v, dans lequel la poudre est sélectionnée parmi des graines finement broyées, des grains de céréales ou un régime à base de grains de céréales finement broyés pour rongeurs
- au moins un agent de masquage de l'arôme ;
- un composé bioactif.

2. Formulation de pilule semi-solide selon la revendication 1, dans laquelle les graines finement broyées sont sélectionnées parmi au moins une entre les graines de tournesol et les graines de l'alpiste des Canaries.

3. Formulation de pilule semi-solide selon l'une quelconque des revendications 1 et 2, dans laquelle le régime à base de grains de céréales finement broyés pour rongeurs comprend au moins un parmi de la farine de soja décortiquée, des rebuts de blé, des flocons de maïs, du maïs broyé, de la farine de poisson, de la mélasse de canne, du blé broyé, du lactosérum séché, de l'huile de soja et de la levure de bière séchée.

4. Formulation de pilule semi-solide selon l'une quelconque des revendications 1 à 3, dans laquelle la formulation de pilule semi-solide comprend en outre un conservateur.

5. Formulation de pilule semi-solide selon l'une quelconque des revendications 1 à 4, dans laquelle l'agent épaississant est de la gélatine en une concentration comprise entre 5 et 20 % m/v.

6. Formulation de pilule semi-solide selon l'une quelconque des revendications 1 à 5, dans laquelle l'agent épaississant est de l'agar en une concentration comprise entre 1 et 5 % m/v.

7. Formulation de pilule semi-solide selon l'une quelconque des revendications 1 à 6, dans laquelle l'agent épaississant est du carraghénane en une concentration comprise entre 0,02 et 3 % m/v.

8. Formulation de pilule semi-solide selon l'une quelconque des revendications 1 à 7, dans laquelle l'agent épaississant est de l'alginate de sodium en une concentration comprise entre 0,7 et 2 % m/v.

9. Formulation de pilule semi-solide selon l'une quelconque des revendications 1 à 8, dans laquelle l'agent épaississant est de la pectine en une concentration comprise entre 0,5 et 5 % m/v.

10. Formulation de pilule semi-solide selon l'une quelconque des revendications 1 à 9, dans laquelle l'édulcorant non digestible est du sucralose en une concentration comprise entre 1 et 5 % m/v.

11. Formulation de pilule semi-solide selon l'une quelconque des revendications 1 à 10, dans laquelle l'édulcorant non digestible est de la saccharine de calcium en une concentration comprise entre 0,1 et 0,25 % m/v.

12. Formulation de pilule semi-solide selon l'une quelconque des revendications 1 à 11, dans laquelle l'agent de masquage de l'arôme est sélectionné parmi la vanille, la fraise, la myrtille et le chocolat.

13. Formulation de pilule semi-solide décrite dans les revendications 1 à 12, destinée à être utilisée dans l'administration orale de médicaments et de composés bioactifs à des rongeurs.

14. Formulation de pilule semi-solide décrite dans les revendications 1 à 12, destinée à être utilisée dans des méthodes de dépistage toxicologique des rongeurs.

15. Formulation de pilule semi-solide décrite dans les revendications 1 à 12, destinée à être utilisée dans la production d'appâts toxiques pour rongeurs.
